# FASCICULE DE BREVET EUROPEEN

(11) **EP 1 631 322 B1**
(45) Date de publication et mention de la délivrance du brevet: **01.08.2007**
(21) Numéro de dépôt: 04741731.6
(22) Date de dépôt: 04.06.2004
(51) Int. Cl.: A61L 2/02, A61L 2/26, A61L 2/18

(54) **DISPOSITIF GENERATEUR DE CAVITATION POUR NETTOYAGE, STERILISATION ET DESINFECTION**
KAVITATIONSERZEUGENDE VORRICHTUNG ZUM REINIGEN, STERILISIEREN UND DESINFIZIEREN
CAVITATION GENERATING DEVICE FOR CLEANING, STERILIZING AND DISINFECTING

(30) Priorité: 06.06.2003 EP 03012883
(43) Date de publication de la demande: 08.03.2006
(73) Titulaire: Rossell, Jordi, 1092 Belmont-sur-Lausanne (CH)
(72) Inventeur: Rossell, Jordi, 1092 Belmont-sur-Lausanne (CH)
(74) Mandataire: Wenger, Joel-Théophile
(86) Numéro de dépôt international: PCT/EP2004/051025
(87) Numéro de publication internationale: WO 2004/108169

(56) Documents cités:
- WO-A-00/45859
- WO-A-95/35069
- US-A- 4 511 254
- US-A- 5 183 513
- US-A- 5 519 670

## Description

La présente invention concerne un dispositif générant un effet de cavitation dans un liquide contenu dans un récipient. Cet effet est utilisé notamment pour la stérilisation, la désinfection ou le traitement d'objets immergés dans le liquide.

Plus particulièrement, l'invention concerne un dispositif apte à créer dans un liquide des variations de pression dont la forme et l'amplitude permet d'engendrer la cavitation dans ce liquide.

La cavitation est un phénomène thermodynamique entraînant la formation et l'implosion de bulles de vapeur (ou cavités) dans un liquide lorsque, et à l'endroit où, ce liquide est soumis à de brusques variations de pression à une température donnée. Ces bulles implosent en produisant des dégagements d'ondes et de chaleur ponctuels qui provoquent des réactions sur le liquide lui-même, sur les parois du récipient ou sur tout objet qui serait immergé dans le liquide.

Ce phénomène de cavitation est surtout connu pour ses effets indésirables dans les systèmes hydrauliques, tels que la corrosion des surfaces, le bruit et la perte d'appui avec le liquide. Malgré ces inconvénients, la cavitation peut présenter des aspects positifs dans certaines applications. En effet, ses particularités mécaniques, thermiques et chimiques peuvent être exploitées avec profit pour le nettoyage, la stérilisation ou le traitement d'objets. L'implosion des bulles de vapeur consécutive à la chute de pression sur une surface ou sur une impureté en dessous de la tension de vapeur d'un liquide libère une onde thermique ponctuelle en un temps très court et sur une surface très petite, et par conséquent provoque une grande élévation de température très localisée. De plus, les réactions chimiques, voire photo-luminescentes, induites par cet accroissement de température sont non négligeables. Ainsi, cette conjonction d'effets mécaniques, thermiques et chimiques contribue grandement à une action efficace du liquide comme agent de stérilisation ou de traitement des objets qui y sont immergés ou du récipient lui-même contenant le liquide.

Plusieurs documents de l'état de la technique décrivent des applications de la cavitation et des moyens pour l'obtenir:
- les documents US5432969, GB2273926, DE3903648 ou US4007007 développent des dispositifs de mise en circulation turbulente d'un liquide dans le but de le stériliser.
- le document US3740028 décrit un dispositif où la cavitation est générée par des variations de volume d'une enceinte de traitement par différents procédés mécaniques ou électromécaniques. Des vibrations sonores sont transmises à une membrane fermant une enceinte de traitement.
- les documents EP0766535 US4993947 décrivent un dispositif dans lequel le récipient à traiter ou des objets contenus dans le récipient sont placés sur le passage d'un flux de liquide cavitant créé au moyen de pompes.
- EP0521119 et EP1146914 concernent des dispositifs de mise en résonance d'une colonne de liquide créant une suite de dépression et de remise en pression dans une enceinte apte à engendrer la cavitation dans cette enceinte.

Le dernier document EP1146914 décrit un dispositif de nettoyage et de stérilisation à l'intérieur d'une enceinte qui comprend une alimentation en liquide de stérilisation et des moyens pour induire au sein de ce liquide des variations de pression ayant une amplitude, une fréquence et une pente adéquate pour engendrer de la cavitation. Ces moyens comprennent une colonne de liquide entre ladite enceinte et un organe de commutation. Ce dernier permet de relier périodiquement la colonne de liquide à deux niveaux de pression déterminés. L'écart entre ces niveaux correspond à l'amplitude désirée de la variation de pression et la transition rapide d'un niveau à l'autre, qui est effectuée par le commutateur, correspond à une pente suffisamment raide pour créer la cavitation. Dans ce dispositif, le bas niveau de pression est créé au moyen d'une pompe à vide et le haut niveau équivaut à la pression atmosphérique.

Ce dispositif présente plusieurs inconvénients dus d'une part à diverses faiblesses mécaniques du commutateur et d'autre part à la présence de la source de basse pression reliée à une pompe à vide.

En effet, le commutateur comporte des pièces en rotation soumises à des frottements variables relativement importants. L'usure qui en résulte nuit à l'étanchéité de l'ensemble du commutateur dont les performances peuvent ainsi se dégrader. Les ouvertures sur le tube axial en rotation qui relient alternativement les conduits amenant la basse et la haute pression à la colonne de liquide se ferment et s'ouvrent progressivement lors de chaque cycle dépression - mise en pression. Cette transition progressive est liée aux contraintes de construction mécanique qui imposent des dimensions, une forme et une disposition particulière à ces ouvertures. Idéalement, l'ouverture et la fermeture de ces ouvertures devraient être brusques et sans transition pour une performance optimale du commutateur. De plus, lors de la rotation de l'axe, on observe une phase momentanée pendant laquelle les deux conduits sont fermés car les ouvertures sur l'axe du commutateur se trouvent en opposition par rapport aux conduits. Ce phénomène accentue l'usure et les pertes mécaniques dues aux frottements en perturbant le mouvement de la colonne de liquide.

La source de basse pression constituée par une pompe à vide reliée à un réservoir introduit aussi certaines contraintes notamment par les dimensions importantes du réservoir par rapport au volume de l'enceinte et par la nécessité d'une vidange périodique pouvant occasionner un risque de contamination. La connexion du réservoir au commutateur amène des pertes de charges inévitables qui sont défavorables au rendement de l'ensemble du dispositif.

La pompe à vide de dimensions relativement importantes a une durée de vie limitée par la condensation due lors de la phase de détente (retour à la pression atmosphérique). La connexion de la pompe avec le réservoir amène d'autres pertes de charge qui s'ajoutent à celles de la connexion au commutateur.

Le but de la présente invention est de pallier les inconvénients du dispositif de l'art antérieur décrit plus haut à savoir:
- diminuer les frottements et les pertes de charges et par conséquent l'usure prématurée des pièces mécaniques du dispositif.
- supprimer la phase de fermeture totale des conduits pendant le cycle de variation de pression
- accentuer et stabiliser le passage de la basse pression à la pression atmosphérique.
- permettre le fonctionnement en continu.

Ce but est atteint par un dispositif générateur de cavitation servant au nettoyage, à la stérilisation ou au traitement d'objets à l'intérieur d'une enceinte ou de l'enceinte elle-même comprenant une alimentation de ladite enceinte en liquide approprié et des moyens pour induire au sein dudit liquide des variations de pression ayant une amplitude, une fréquence et une pente aptes à engendrer la cavitation dans ce liquide, lesdits moyens comprenant une colonne de liquide reliant ladite enceinte à une première flasque d'une pompe comportant un compartiment de forme généralement cylindrique dans lequel coulisse un piston entraîné par un groupe moteur dans un mouvement de va et vient entre ladite première flasque et une seconde flasque munie d'un joint d'étanchéité pour le passage de l'axe du piston, lesdites flasques fermant le compartiment de la pompe à chaque extrémité, caractérisé en ce que
ledit piston est muni d'au moins une soupape avec des ouvertures se fermant dans une première phase du mouvement dudit piston, appelée aspiration, et s'ouvrant dans une seconde phase du mouvement dudit piston, appelée détente, créant une succession périodique de dépressions et de remises en pression du liquide contenu dans l'enceinte,
et en ce que la seconde flasque de la pompe comporte deux ouvertures équipées chacune de soupapes servant à contrôler respectivement la sortie d'air lors de la phase d'aspiration et l'entrée d'air lors de la phase de détente.

L'avantage principal du dispositif selon l'invention est l'absence de la pompe à vide et de son réservoir associé faisant office de source de basse pression constante. Le commutateur est remplacé par une pompe à piston créant alternativement une dépression et le retour à la pression atmosphérique dans le compartiment connecté à la colonne de liquide. Les inconvénients liés à la présence d'une pompe à vide séparée et ceux liés au commutateur sont ainsi surmontés.

Si la forme préférée de l'invention pour le piston et le compartiment de la pompe est le cylindre, la présente invention couvre également d'autres formes telles que l'ovale par exemple. Il semble évident pour des problèmes d'étanchéité que cette forme doit être continue c'est-à-dire sans arête. D'autres systèmes engendrant une variation cyclique du volume d'une chambre peuvent aussi être envisagés pour générer la cavitation.

Dans ce dispositif, les variations de pression sont induites dans l'enceinte grâce au mouvement du piston de la pompe qui, dans un sens, aspire le liquide hors du compartiment qui s'échappe par l'ouverture ad hoc de la flasque. Dans le sens inverse, le retour à la pression atmosphérique s'effectue grâce à l'ouverture des soupapes du piston et de la soupape de l'entrée d'air de la flasque.

L'invention sera mieux comprise grâce à la description détaillée qui va suivre et qui se réfère aux dessins annexés qui sont donnés à titre d'exemple nullement limitatif, dans lesquels:
- la figure 1 représente un schéma bloc du dispositif selon l'invention avec une enceinte reliée à la pompe par une conduite.
- la figure 2 représente une variante du schéma bloc de la figure 1 où la conduite joue le rôle de l'enceinte.
- la figure 3 illustre une coupe de la pompe en phase d'aspiration
- la figure 4 illustre une coupe de la pompe en phase de détente

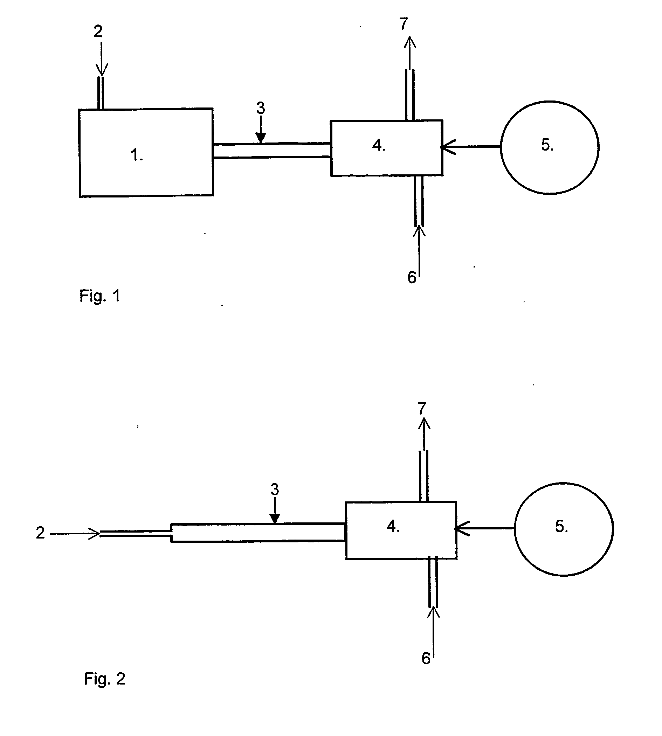

Le schéma bloc de la figure 1 montre l'ensemble du dispositif selon l'invention comprenant une enceinte (1), une conduite (3) reliant une pompe (4) à ladite enceinte (1). Un groupe moteur (5) actionne la pompe (4) qui induit des variations de pression dans l'enceinte (1) via la conduite (3).

L'enceinte (1) constituant le volume de nettoyage, de stérilisation ou de traitement comporte une entrée de liquide de traitement (2) raccordée à un tuyau aboutissant à un récipient (non représenté). Au cours du processus, la pompe aspire le liquide de traitement dans l'enceinte qui ensuite transite par la conduite (3) vers la pompe (4). Cette dernière comporte un échappement (7) servant à évacuer l'air et le liquide lors de l'aspiration et une entrée d'air (6) servant à la remise en pression.

La première phase du cycle se distingue par la création de bulles de vapeur dans l'enceinte (1) due à la baisse subite de la pression. Le niveau de cette dépression nécessaire à la formation des bulles dépend de la température, de la nature et de la pureté du liquide. Chaque impureté ou discontinuité mécanique sera un germe de bulle potentiel pour un même liquide. Les conditions de cette première phase sont obtenues par le déplacement du piston de la pompe (4) reliée à l'enceinte (1) de liquide. La dimension de l'entrée de liquide (2) est suffisamment petite pour que la perte de charge qu'elle crée en aval de l'augmentation de volume du système clos et rigide entraîne l'abaissement de sa pression interne.

La seconde phase est l'implosion des bulles de vapeur obtenue par un retour instantané de la pression atmosphérique dans l'enceinte (1) au moyen d'une soupape de la pompe (4) qui permet la connexion de la conduite (3) directement à l'entrée d'air (6) dès que le piston amorce sa course au début de la phase de détente. La remise en pression en amont de la conduite permet un retour du liquide contenu dans ladite conduite vers l'enceinte (1) et crée une surpression momentanée qui permet l'implosion simultanée et complète de toutes les bulles de vapeur précédemment formées. Ce phénomène est connu sous le nom de "coup de bélier" et son caractère périodique fait qu'il participe ensuite à un nouvel abaissement de la pression dans l'enceinte (1) afin de retrouver la première phase et de continuer le cycle.

La figure 2 montre une variante dans laquelle l'enceinte (1) joue le rôle de la conduite (3) et vice-versa. Cette configuration est possible dans le cas où ses dimensions présenteraient une fréquence propre adaptable à celle de la pompe (4) comme dans le cas de la conduite (3) reliant l'enceinte (1) de la figure précédente.

L'effet maximum du changement d'état, c'est-à-dire le passage du liquide au gaz, dans une même enceinte (1) est proportionnel à la dépression créée par la pompe (4) mais aussi à la masse du mélange (air et liquide) contenu dans la conduite (3) et à l'ajustage de la fréquence du cycle sur la fréquence propre de cette dernière.

Le remplissage du système est assuré par l'entrée de liquide (2) dans l'enceinte (1). Le débit de celle-ci doit être suffisant pour que la conduite (3) reste constamment amorcée mais ni trop important afin de ne pas compromettre la valeur de la dépression obtenue.

La vitesse de rotation du moteur (5) entraînant la pompe (4) peut être ajustable en fonction de la nature du liquide utilisé, de sa température et de celle de l'enceinte (1). L'état du mélange extrait de la cavité traitée (proportion de gaz dissout) au travers de la conduite (3) joue aussi un rôle et peut exiger une adaptation en particulier lors d'une opération de dissolution de matière présente dans l'enceinte. Un asservissement de la vitesse de rotation du moteur par une boucle de réglage serait donc nécessaire pour une optimisation du fonctionnement. La consigne est donnée par un capteur de la pression instantanée dans l'enceinte. Une vanne proportionnelle sur l'entrée de liquide pourra aussi utiliser cette valeur pour ajuster son ouverture.

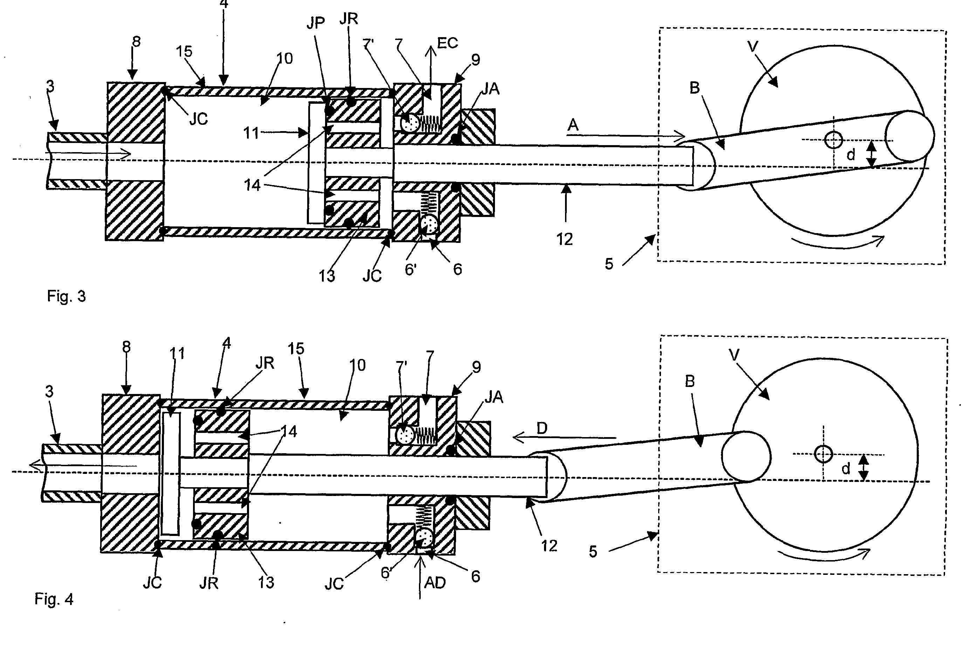

Les figures 3 et 4 montrent une coupe d'un exemple de pompe à piston durant les phases d'aspiration et de détente (A, D). La pompe (4) comprend un compartiment (10) formé par deux flasques (8, 9) reliées par une paroi cylindrique (15) de manière étanche au moyen de joints (JC) du type O-ring. La première flasque (8) comporte une ouverture axiale reliée à la conduite (3) aboutissant à l'enceinte. La seconde flasque (9) est munie d'une ouverture axiale, rendue étanche avec un joint (JA), servant de passage à l'axe (12) du piston (11) et de deux soupapes (6', 7') contrôlant respectivement l'entrée d'air (6) et la sortie de mélange air et liquide (7) ou échappement.

L'intérieur du compartiment (10) est parcouru par un piston (11) solidaire d'un axe (12) qui le relie au groupe moteur (5). Le piston (11) est en outre associé à une soupape (13) munie d'un joint radial (JR) qui assure d'une part l'étanchéité du piston (11) et d'autre part la commande d'ouverture et de fermeture de la soupape (13) par son frottement contre la paroi (15) interne du compartiment (10) de la pompe (4) lors du mouvement de va et vient du piston (11).

Lors de la phase d'aspiration illustrée par la figure 3, le piston (11) s'éloigne de la flasque (8) connectée à la conduite (3) suivant la flèche (A). Les ouvertures (14) de la soupape (13) sont fermées hermétiquement par le piston (11) à l'aide d'un joint (JP) s'appliquant contre la face arrière du piston (11). L'air et le liquide contenus dans le compartiment (10) s'évacuent à travers l'ouverture d'échappement (7) de la flasque (9) suivant la flèche EC; la soupape d'échappement (7') étant ouverte et celle d'entrée (6') étant fermée sous la pression du mélange d'air et de liquide sortant. La dépression ainsi créée dans le compartiment (10) de la pompe se transmet à l'enceinte (1) via la colonne de liquide dans la conduite (3). A la fin de la phase d'aspiration, le piston (11) et la soupape (13) atteignent la flasque opposée (9) de la pompe (4) comportant les soupapes d'entrée et de sortie d'air (6', 7'). A ce moment, la course du piston (11) s'inverse provoquant un brusque retour à la pression atmosphérique par l'ouverture simultanée de la soupape (13).

La figure 4 illustre cette phase de retour ou de détente (flèche D) où les ouvertures (14) de la soupape (13) du piston (11) se dégagent par l'action simultanée de l'avancement du piston (11) et du frottement du joint radial (JR) contre la paroi interne du compartiment (10) de la pompe (4) qui retient la soupape (13). De plus, la soupape d'admission (6') de l'entrée d'air (6) s'ouvre pour laisser pénétrer l'air dans le compartiment (10) de la pompe (4) suivant la flèche AD, tandis que la soupape (7') de l'échappement (7) se ferme par l'aspiration de l'air entrant. Cette augmentation de la pression est immédiatement transmise à la colonne de liquide dans la conduite (3) puis à l'enceinte (1). Lorsque le piston (11) atteint la flasque (8) du côté conduite (3), à la fin de sa course, le liquide supplémentaire aspiré précédemment passe à travers la soupape (13) et un nouveau cycle d'aspiration - détente recommence.

Le groupe moteur (5) chargé du mouvement alternatif du piston (11) de la pompe (4) comprend un volant d'inertie (V) et une bielle- manivelle (B) reliée mécaniquement à l'axe (12) du piston. Un décentrage (d) de l'axe de rotation du moteur par rapport à l'axe du piston influence la forme de la variation du mouvement du piston et peut favoriser la rapidité de la remise en pression.

Les dimensions et la rigidité de la conduite (3) reliant l'enceinte (1) à la pompe (4) sont en relation directe avec la fréquence du cycle qui est proportionnelle à la vitesse de rotation du moteur.

Par exemple, une conduite métallique en cuivre 10mm de diamètre intérieur et 1mm de paroi pour une longueur de 320mm a donné de bons résultats à une fréquence avoisinant les 15Hz avec la plupart des liquides utilisés. Ce dimensionnement convient pour générer un régime de cavitation convenable dans des volumes allant jusqu'à quelques litres. Le diamètre de l'entrée de liquide frais dans l'enceinte par rapport au volume de celle-ci est un compromis entre un bon remplissage de l'ensemble enceinte - conduite et la perte inhérente du niveau de dépression. Une ouverture de 3mm prolongée par un tube en PVC souple de 4mm de diamètre intérieur pour une longueur de 1m s'est révélé un choix efficace. Le débit de fonctionnement dépendant de la nature du liquide utilisé est dans cet exemple de l'ordre de 1 l/min.

L'enceinte de traitement peut être constituée de plusieurs parties assemblées de manière amovible et étanche l'une par rapport à l'autre. Ce type de configuration permet d'adapter le volume de l'enceinte aux objets introduits afin d'optimiser la formation des bulles.

La forme et la disposition de l'enceinte par rapport à la conduite et à la pompe sont des facteurs qui jouent un rôle surtout lors de l'évacuation des bulles d'air résiduelles lors de la phase d'aspiration.

Par exemple, une disposition verticale de l'ensemble du dispositif sera préférée avec une pompe et son groupe moteur situés au-dessus d'une l'enceinte, la pompe étant reliée par une conduite verticale à ladite enceinte. Le mouvement du piston s'effectuerait ainsi de bas en haut et inversement. Si la pompe se trouve au-dessous de l'enceinte une conduite " en U " pourrait être utilisée.

L'utilisation d'une pompe à piston comme générateur de dépression s'est montrée la plus performante par rapport à d'autres types de pompes car le piston prolonge et épouse la colonne de liquide qu'il entraîne. En ce qui concerne la soupape associée au piston, d'autres variantes peuvent être utilisées pour obtenir une transition dépression - remise en pression. L'exemple choisi et schématisé dans les figures 3 et 4 s'est révélé être un des plus simple et des plus efficace lors des essais.

## Revendications

1. Dispositif générateur de cavitation servant au nettoyage, à la stérilisation ou au traitement d'objets à l'intérieur d'une enceinte (1) ou de l'enceinte elle-même comprenant une alimentation (2) de ladite enceinte (1) en liquide approprié et des moyens pour induire au sein dudit liquide des variations de pression ayant une amplitude, une fréquence et une pente aptes à engendrer la cavitation dans ce liquide, lesdits moyens comprenant une colonne de liquide (3) reliant ladite enceinte (1) à une première flasque (8) d'une pompe (4) comportant un compartiment (10) de forme généralement cylindrique dans lequel coulisse un piston (11) entraîné par un groupe moteur (5) dans un mouvement de va et vient entre ladite première flasque (8) et une seconde flasque (9) munie d'un joint d'étanchéité (JA) pour le passage de l'axe (12) du piston (11), lesdites flasques (8, 9) fermant le compartiment (10) de la pompe (4) à chaque extrémité, **caractérisé en ce que**
ledit piston (11) est muni d'au moins une soupape (13) avec des ouvertures (14) se fermant dans une première phase du mouvement dudit piston (11), appelée aspiration (A), et s'ouvrant dans une seconde phase du mouvement dudit piston (11), appelée détente (D), créant une succession périodique de dépressions et de remises en pression du liquide contenu dans l'enceinte (1),
et **en ce que** la seconde flasque (9) de la pompe (1) comporte deux ouvertures (6, 7) équipées chacune de soupapes (6', 7') servant à contrôler respectivement la sortie d'air (EC) lors de la phase d'aspiration (A) et l'entrée d'air (AD) lors de la phase de détente (D).

2. Dispositif selon la revendication 1 **caractérisé en ce que** la soupape (13) du piston (11) est munie d'un joint radial (JR) assurant d'une part l'étanchéité du piston (11) et d'autre part la commande de fermeture et de dégagement des ouvertures (14) de ladite soupape (13) par le frottement dudit joint (JR) contre la paroi (15) interne de la pompe (4) lors du mouvement de va et vient du piston (11).

3. Dispositif selon les revendications 1 et 2 **caractérisé en ce que** le piston (11) s'éloigne de la première flasque (8) reliée à la colonne de liquide (3) lors de la phase d'aspiration (A), les ouvertures (14) de la soupape (13) associée audit piston (11) étant fermées hermétiquement au moyen d'un joint (JP), ladite phase d'aspiration (A) crée une dépression dans le compartiment (10) de la pompe (4) et l'air étant évacué à travers la soupape (7') d'échappement de la seconde flasque (9), ladite dépression étant transmise à la colonne de liquide (3) dans la conduite puis à l'enceinte (1).

4. Dispositif selon les revendications 1 et 2 **caractérisé en ce que** les ouvertures (14) de la soupape (13) associée audit piston (11) se dégagent par l'action simultanée de l'avancement du piston (11) et du frottement du joint radial (JR) contre la paroi (15) interne de la pompe (4) retenant ladite soupape (13) lors de la phase de détente (D), succédant immédiatement à la phase d'aspiration (A) après que le piston (11) a atteint la seconde flasque (9), ladite phase de détente (D) crée un retour à la pression atmosphérique dans le compartiment (10) de la pompe (4), l'air pénétrant à travers la soupape (6') d'entrée de la seconde flasque (9), cette augmentation de pression étant transmise à la colonne de liquide (3) dans la conduite puis à l'enceinte (1).

5. Dispositif selon les revendications 1 à 4 **caractérisé en ce que** le piston (11) recommence un nouveau cycle d'aspiration (A) - détente (D) chaque fois lorsqu'il atteint la première flasque (8) du compartiment (10) de la pompe (4) induisant les variations de pression nécessaires à la génération de la cavitation dans l'enceinte (1).

6. Dispositif selon la revendication 1 **caractérisé en ce que** l'enceinte (1) est constituée de plusieurs parties assemblées de manière amovible et étanche l'une par rapport à l'autre.

7. Dispositif selon la revendication 1 **caractérisé en ce que** l'enceinte (1) est constituée par la conduite contenant la colonne de liquide (3).

8. Dispositif selon la revendication 1 **caractérisé en ce que** la fréquence du cycle aspiration (A) - détente (D) de la pompe (4), proportionnelle à la vitesse de rotation du groupe moteur (5), est adaptée au dimensionnement de la conduite et/ou du volume de l'enceinte (1).

## Claims

1. Cavitation generating device for cleaning, sterilizing or treating objects inside an enclosure (1) or of the enclosure itself comprising an appropriate liquid supply (2) of said enclosure (1) and means for inducing in the liquid pressure variations having an amplitude, a frequency and a slope able to generate cavitation in the liquid, said means comprising a liquid column (3) linking said enclosure (1) to a first flask (8) of a pump (4) including a compartment of a generally cylindrical shape in which slides a piston (11) driven by an engine group in a back and forward motion between said first flask (8) and a second flask (9) provided with a watertight joint (JA) for the passage of the axis (12) of the piston (11), said flasks (8, 9) closing the compartment (10) of the pump (4) at each extremity, **characterized in that**
said piston (11) includes at least one valve (13) with apertures (14) closing in a first phase of the motion of said piston, called suction (A), and opening in a second phase of the motion of said piston (11), called expansion (D), creating a periodic succession of depressions and returns to pressure of the liquid in the container (1),
and **in that** the second flask of the pump (1) includes two apertures (6, 7) each provided with valves (6', 7') for controlling respectively the air output during the suction phase (A) and the air input during the expansion phase (D).

2. Device according to claim 1 **characterized in that** the valve (13) of the piston (11) includes a radial joint (JR) providing on one hand the watertightness of the piston (11) and on the other hand the command of closing and opening the apertures (14) of said valve (13) through friction of said joint (JR) against the internal wall (15) of the pump (4) during the back and forward motion of the piston (11).

3. Device according to claims 1 and 2 **characterized in that** the piston (11) moves away from the first flask (8) linked to the liquid column (3) during the suction phase (A), the apertures (14) of the valve (13) associated to said piston (11) being hermetically closed by means of a joint (JP), said suction phase (A) creates a depression in the compartment (10) of the pump (4) and the air being evacuated through the exhaust valve (7') of the second flask (9), said depression being transmitted to the liquid column (3) in the pipe then to the enclosure (1).

4. Device according to claims 1 and 2 **characterized in that** the apertures (14) of the valve (13) associated to said piston (11) open by the simultaneous action of the forward motion of the piston (11) and the friction of the radial joint (JR) against the internal wall of the pump (4) retaining said valve (13) during the expansion phase (D), succeeding immediately to the suction phase (A) after the piston (11) has reached the second flask (9), said expansion phase (D) creates a return to the atmospheric pressure in the compartment (10) of the pump (4), the air penetrating through the input valve (6') of the second flask (9), this pressure increase being transmitted to the liquid column (3) in the pipe then to the enclosure (1).

5. Device according to claims 1 to 4 **characterized in that** the piston (11) starts again a new cycle of suction (A) - expansion (D) each time it reaches the first flask (8) of the compartment (10) of the pump (4) inducing the pressure variations necessary for generating the cavitation in the enclosure (1).

6. Device according to claim 1 **characterized in that** the enclosure (1) is made of several parts assembled in a removable way and watertight one relative to the other.

7. Device according to claim 1 **characterized in that** the enclosure (1) is made of the pipe containing the liquid column (3).

8. Device according to claim 1 **characterized in that** the frequency of the cycle of suction (A) - expansion (D) of the pump (4), proportional to the rotating speed of the engine group (5), is adapted to the dimensions of the pipe and/or to the volume of the enclosure (1).

## Patentansprüche

1. Zur Reinigung, Sterilisation oder Behandlung von Gegenständen im Inneren einer Kammer (1) oder der Kammer selbst dienende Kavitationsgeneratorvorrichtung mit einer Speisung (2) der Kammer (1) mit geeigneter Flüssigkeit und mit Mitteln, um im Inneren der Flüssigkeit Druckschwankungen zu erregen, die eine Amplitude, Frequenz und Steigung besitzen, die in der Lage sind, Kavitation in dieser Flüssigkeit zu erzeugen, wobei die Mittel eine Flüssigkeitssäule (3) umfassen, die die Kammer (1) mit einem ersten Flansch (8) einer Pumpe (4) verbindet, die ein allgemein zylindrisches Abteil (10) aufweist, in dem ein Kolben (11) gleitet, der von einer Antriebsgruppe (5) zu einer Hin- und Herbewegung zwischen dem ersten Flansch (8) und einem zweiten Flansch (9) angetrieben wird, der mit einer Dichtung (JA) für den Durchlass der Achse (12) des Kolbens (11) versehen ist, wobei die Flansche (8, 9) das Abteil (10) der Pumpe (4) an den beiden Enden verschliessen, **dadurch gekennzeichnet, dass**
der Kolben (11) mit zumindest einem Ventil (13) mit Öffnungen (14) versehen ist, die sich in einer ersten, Ansaugen (A) genannten Bewegungsphase des Kolbens (11) schliessen und in einer zweiten, Entspannung (D) genannten Bewegungsphase des Kolbens (11) öffnen, wodurch eine periodische Folge von Depressionen und Kompressionen der in der Kammer (1) enthaltenen Flüssigkeit erzeugt wird,
und **dadurch**, dass der zweite Flansch (9) der Pumpe (1) zwei Öffnungen (6, 7) aufweist, deren jede mit Ventilen (6', 7') ausgerüstet ist, die dazu dienen, das Entweichen von Luft (EC) während der Phase des Ansaugens (A) und den Eintritt von Luft (AD) während der Phase der Entspannung (D) zu steuern.

2. Vorrichtung nach Anspruch 1, **dadurch gekennzeichnet, dass** das Ventil (13) des Kolbens (11) mit einer Radialdichtung (JR) versehen ist, die während der Hin- und Herwegung des Kolbens (11) einerseits die Abdichtung des Kolbens (11) und andererseits die Steuerung des Schliessens und der Freilegung der Öffnungen (14) des Ventils (13) durch Reibung der Dichtung (JR) an der Innenwand (15) der Pumpe gewährleistet.

3. Vorrichtung nach Ansprüchen 1 und 2, **dadurch gekennzeichnet, dass** der Kolben (11) sich während der Ansaugphase (A) von dem ersten Flansch (8) entfernt, der mit der Flüssigkeitssäule (3) verbunden ist, wobei die Öffnungen (14) des mit dem Kolben (11) verbundenen Ventils (13) durch eine Dichtung (JP) dicht verschlossen werden, die Ansaugphase (A) einen Unterdruck in der Kammer (10) der Pumpe (4) erzeugt und die Luft durch das Austrittsventil (7') des zweiten Flansches (9) abgeführt wird, während der Unterdruck an die Flüssigkeitssäule (3) im Kanal und von da an die Kammer (1) übertragen wird.

4. Vorrichtung nach Ansprüchen 1 und 2, **dadurch gekennzeichnet, dass** die Öffnungen (14) des mit dem Kolben (11) verbundenen Ventils (13) durch die gleichzeitige Wirkung des Vorrückens des Kolbens (11) und der Reibung der Radialdichtung (JR) an der Innenwand (15) der Pumpe (4) freigegeben werden, durch die das Ventil (13) während der Entspannungsphase (D) zurückgehalten wird, die unmittelbar auf die Ansaugphase (A) folgt, nachdem der Kolben (11) den zweiten Flansch (9) erreicht hat, die Entspannungsphase (D) eine Rückkehr zum Atmosphärendruck in der Kammer (10) der Pumpe (4) erzeugt, wobei Luft durch das Eintrittsventil (6') des zweiten Flansches (9) eindringt und dieser Druckanstieg an die Flüssigkeitssäule (3) im Kanal und von da an die Kammer (1) übertragen wird.

5. Vorrichtung nach Ansprüchen 1 bis 4, **dadurch gekennzeichnet, dass** der Kolben (11) jedesmal, wenn er den ersten Flansch (8) der Kammer (10) der Pumpe (4) erreicht hat, einen neuen Zyklus von Ansaugen (A) und Entspannen (D) beginnt, wodurch die Druckschwankungen verursacht werden, die für die Erzeugung der Kavitation in der Kammer (1) erforderlich sind.

6. Vorrichtung nach Anspruch 1, **dadurch gekennzeichnet, dass** die Kammer (1) aus mehreren beweglich und gegeneinander dicht zusammengefügten Teilen besteht.

7. Vorrichtung nach Anspruch 1, **dadurch gekennzeichnet, dass** die Kammer (1) aus dem Kanal besteht, der die Flüssigkeitssäule (3) enthält.

8. Vorrichtung nach Anspruch 1, **dadurch gekennzeichnet, dass** die der Drehbewegung der Antriebsgruppe (5) proportionale Frequenz der Ansaug- (A) und Entspannungs- (D) zyklen der Pumpe (4) den Abmessungen des Kanals und/oder dem Volumen der Kammer (1) angepasst ist.
